# EUROPEAN PATENT APPLICATION

(11) **EP 2 606 878 A1**
(43) Date of publication of application: **26.06.2013**
(21) Application number: 11195539.9
(22) Date of filing: 23.12.2011
(51) Int. Cl.: A61K 9/16, A61K 9/20, A61K 31/485

(54) **Granulate and controlled release dosage forms comprising a hygroscopic active ingredient**

(71) Applicant: Hexal AG, 83607 Holzkirchen (DE)
(72) Inventor: Wiedmann, Markus, 83607 Holzkirchen (DE)
(74) Representative: Wibbelmann, Jobst

(57) **Abstract**

The present invention relates to granulate comprising a hygroscopic active ingredient, particularly hydromorphone, or a salt thereof, methods for preparing it and controlled release dosage forms for oral administration comprising said granulate.

## Description

The present invention relates to granulate comprising a hygroscopic active ingredient or a salt thereof, methods for preparing it and controlled release dosage forms for oral administration comprising said granulate.

### Background prior art

Hydromorphone (CAS Registry Number 466-99-9; IUPAC name: 4,5-α-epoxy-3-hydroxy-17-methyl morphinan-6-one) is a potent centrally-acting analgesic drug of the opioid class. It is a semi-synthetic derivative of morphine used for the treatment of moderate to severe pain. Methamphetamine (CAS Registry Number 537-46-2; IUPAC name: N-methyl-1-phenylpropan-2-amine) is used in the treatment of attention-deficit hyperactivity disorder (ADHD), exogenous obesity and narcolepsy. Etoricoxib (CAS Registry Number 202409-33-4; IUPAC name: 5-chloro-6'-methyl-3-[4-(methylsulfonyl)phenyl]-2,3'-bipyridine) is a COX-2 selective inhibitor. It is used in the treatment of rheumatoid arthritis, psoriatic arthritis, osteoarthritis, ankylosing spondylitis, chronic low back pain, acute pain and gout.

Different medicinal products containing immediate release and controlled release formulations of hydromorphone, etoricoxib, and methamphetamine are well known. To obtain controlled release formulations or controlled release dosage forms, the ingredients are typically processed with release controlling polymers, e.g. with an osmotically retarding polymer, to form a controlled release dosage system. One typical controlled release dosage system using osmotically retarding polymers is OROS (Osmotic-controlled Release Oral delivery System). This is a controlled release oral drug delivery system in the form of a tablet. The tablet has a water-permeable jacket with one or more small holes. As the tablet passes through the body, the osmotic pressure of water entering the tablet pushes the active ingredient out through the opening in the tablet.

However, ingredients such as hydromorphone, etoricoxib, and methamphetamine are very hygroscopic, i.e. they absorb moisture from the air even under normal room conditions. This results in the major drawback that e.g. during the granulation of the ingredients with the release controlling hydrophilic polymers the polymers start to swell. In addition, the obtained granulate tends to agglutinate and agglomerate, which complicates the compressing into tablets.

US 5273758 discloses the use of polyethylene oxide polymer as a directly compressible binder matrix for therapeutically active dosage forms.

EP 0769949 B1 discloses the granulation of hydromorphone and polyethylene oxide with an ethanolic solution of polyvinylpyrrolidone, wherein the polyvinylpyrrolidone is used as a binder. However, the use of organic solvents should in general be avoided in the preparation of pharmaceutical dosage form.

Further known are controlled release dosage forms in the form of 4 mg, 8 mg, 16 mg, 32 mg and 64 mg OROS tablets comprising hydromorphone (Jurnista^{®} from Janssen/Cilag). The tablet core is built up of two layers, wherein one of the layers comprises hydromorphone. Lactose is present in the tablet coating in amounts between 0.01 and 10.02 mg depending on the weight of the tablet. In the art of pharmaceuticals, lactose is typically known as filler.

There is a constant search for new granulate comprising hygroscopic active ingredients and release controlling hydrophilic polymers, which can be prepared during a simple granulation process and without the need to use organic solvents. Furthermore, there is a need for granulate comprising hygroscopic active ingredients and release controlling hydrophilic polymers, which does not agglutinate or agglomerate after the granulation. In addition, there is a constant search for granulate comprising hygroscopic active ingredients and release controlling hydrophilic polymers which can easily be compressed into tablets. Furthermore, there is a need for new controlled release dosage forms comprising said granulate. Therefore, it was an object of the present invention to provide granulate comprising hygroscopic active ingredients and release controlling hydrophilic polymers.

It was another object of the present invention to provide an excipient for the granulation of a hygroscopic active ingredient or a salt thereof.

It was a further object of the present invention to provide an excipient for improving the compressibility and/or the flow properties of granulate comprising hygroscopic active ingredients and release controlling hydrophilic polymers.

It was another object of the present invention to provide a method for preparing granulate comprising hygroscopic active ingredients and release controlling hydrophilic polymers.

It was yet another object of the present invention to provide a new controlled release dosage form comprising hygroscopic active ingredients and release controlling hydrophilic polymers and methods for preparing such controlled release dosage forms.

### Summary of the Invention

According to one aspect, the present invention relates to granulate comprising a hygroscopic active ingredient or a salt thereof; 30 to 95 mg of at least one release controlling hydrophilic polymer, in 100 mg of the granulate; and 0.5 to 30 mg lactose or lactose monohydrate, in 100 mg of the granulate.

According to a further aspect of the present invention, there is provided the use of lactose or lactose monohydrate as excipient for the granulation of a hygroscopic active ingredient or a salt thereof.

According to another aspect of the present invention, there is provided the use of lactose or lactose monohydrate for improving the compressibility and/or the flow properties of granulate comprising a hygroscopic active ingredient or a salt thereof, and at least one release controlling hydrophilic polymer.

In still another aspect, there is provided a method for preparing granulate, comprising granulating a hygroscopic active substance or a salt thereof, at least one release controlling hydrophilic polymer, and lactose or lactose monohydrate.

In still another aspect, there is provided a method for preparing granulate, comprising granulating a hygroscopic active substance or a salt thereof, at least one release controlling hydrophilic polymer, and an aqueous lactose or lactose monohydrate solution.

According to a further aspect of the present invention, there is provided granulate obtainable by a method comprising or consisting of granulating a hygroscopic active substance or a salt thereof, at least one release controlling hydrophilic polymer, and lactose or lactose monohydrate.

According to a further aspect of the present invention, there is provided granulate obtainable by a method comprising or consisting of granulating a hygroscopic active substance or a salt thereof, at least one release controlling hydrophilic polymer, and an aqueous lactose or lactose monohydrate solution.

According to another aspect of the present invention, there is provided a controlled release dosage form for oral administration comprising granulate as defined herein.

In still another aspect, there is provided a method for preparing a controlled release dosage form for oral administration, wherein the controlled release dosage form comprises granulate as defined herein.

In yet another aspect of the present invention, there is provided the use of the granulate as defined herein for preparing a controlled release dosage form for oral administration.

The present invention represents an improvement over the prior art as surprisingly the use of lactose or lactose monohydrate as excipient during the granulation of a hygroscopic active substance or a salt thereof and at least one release controlling hydrophilic polymer results in granulate, which may show less tendency to agglutinate or agglomerate. Moreover, the use of lactose or lactose monohydrate as inexpensive excipient and the simple and environmentally friendly granulation method may result in a more economical process. Furthermore, the granulate may exhibit favourable compressibility and flow properties, which may result in a simple method for preparing a controlled release dosage form for oral administration.

### Definitions

The term "hygroscopic active substance" as used herein shall be understood as known in the art. In particular, hygroscopy is the ability of a substance to attract and hold water molecules from the surrounding environment. The hygroscopic active substances are typically stored in sealed containers, preferably under an inert atmosphere. A hygroscopic active substance typically increases in up to about 0.1 to 10 wt.-% after being stored in air for 1 hour.

The term "release controlling hydrophilic polymer" denotes to a hydrophilic polymer, which controls the release of the hygroscopic active substance. In particular, the polymer forms the release controlling matrix, in which the active substance is embedded. Typically, the release controlling hydrophilic polymer is an osmotically retarding polymer.

The term "granulate" as used herein shall be understood as known in the art. In particular, granulate are particles gathered into a larger, permanent aggregate in which the original particles can still be identified. The term granulation refers to the act or process in which primary powder particles are made to adhere to form larger, multiparticle entities called granulate.

The amounts given for the granulate refer to 100 mg of the granulate, wherein the components add up to a maximum of 100 mg. If the sum of the given amounts does not equal 100 mg, the missing amounts are provided by the other explicitly mentioned ingredients, for which no amounts are provided, and/or by any further ingredients, which may be present.

The terms "tablet" and "tablets" as used herein shall be understood as known in the art. In particular, tablets are a solid dosage form. They comprise a mixture of components, usually in powder form, pressed or compacted from a powder or granulate into a solid dose.

The terms "diluents", "lubricants", "colorants" and "glidants" as used herein shall be understood as known in the art.

The term "comprising" as used herein shall in every embodiment, aspect, example, item or claim be interpreted as encompassing all the specifically mentioned features, components or steps as well as optional, additional, unspecified ones. In addition, the term "comprising" as used herein shall also be interpreted to mean "consisting of', therewith defining for every embodiment, aspect, example, item or claim that no further features, components or steps are present.

### Detailed Description of the Invention

### Granulate

According to one aspect the present invention relates to granulate comprising a hygroscopic active ingredient or a salt thereof; 30 to 95 mg of at least one release controlling hydrophilic polymer, in 100 mg of the granulate; and 0.5 to 30 mg lactose or lactose monohydrate, in 100 mg of the granulate. Typically, 100 mg granulate comprises 0.1 to 30 mg of the hygroscopic active ingredient or a salt thereof.

Preferably, the hygroscopic active ingredient is a pharmaceutical ingredient. The hygroscopic active ingredient is typically selected from hydromorphone, hydromorphone salt, methamphetamine, methamphetamine salt, etoricoxib, etoricoxib salt, and any combination thereof, and is particularly hydromorphone or hydromorphone salt.

The hygroscopic active ingredient may be present in the form of a salt, particularly in the form of the hydrochloride, sulfate, trifluoracetate, mucate, oleate, acetate, phosphate, or bitartrate salt, or any combination thereof, and is particularly hydromorphone hydrochloride.

The granulate comprises typically from 50 to 95 mg, particularly from 60 to 95 mg, of at least one release controlling hydrophilic polymer, in 100 mg of the granulate. Furthermore, the at least one release controlling hydrophilic polymer is preferably an osmotically effective polymer. It is typically selected from polyethylene oxide, cellulose ether, hydroxypropyl methylcellulose, hydroxypropyl cellulose, polyvinyl alcohol, methylcellulose, polyvinylpyrrolidone, or any combination thereof, and is preferably polyethylene oxide. The average molecular weight measured by rheological measurements is typically 100.000 to 7.000.000 g/mol, and is preferably about 200.000 to 2.000.000 g/mol.

The lactose is typically present in the form of its monohydrate. Typically, 100 mg of the granulate comprises 0.5 to 25 mg, particularly from 0.5 to 20 mg lactose or lactose monohydrate.

The granulate may further comprise components selected from diluents, lubricants, colorants, glidants, antioxidants, or any combination thereof. The diluents are typically selected from microcrystalline cellulose, calcium phosphate, sodium chloride, sugars, sugar alcohols such as saccharose, sorbitol, mannitol, or any combination thereof. They may be present in 5 to 50 wt.-% of the total granulate. The lubricants are typically selected from magnesium stearate, sodium stearyl fumarate, cetyl alcohol, stearinic acid, fumaric acid, and any combination thereof. They may be present in 0.1 to 10 wt.-% of the total granulate. The colorants are typically selected from ferric oxides. They may be present in 0.01 to 5 wt.-% of the total granulate. The glidants are typically selected from talcum, silicon dioxide, and any combination thereof. They may be present in 0.1 to 10 wt.-% of the total granulate. The antioxidants are typically selected from butylated hydroxytoluene (BHT), ascorbic acid, butylated hydroxyanisole (BHA), and any combination thereof. They may be present in 0.01 to 3 wt.-% of the total granulate.

The granulate may be used for preparing a controlled release dosage form for oral administration.

### Controlled release dosage form

According to a further aspect, the present invention provides a controlled release dosage form for oral administration comprising the granulate as defined herein. The dosage form is typically in the form of a tablet.

Typically, the granulate has been compressed to form the controlled release dosage form, particularly to form the tablet. For compressing, a rotary press or a multi-layer rotary press, such as Fette 1200, Fette 3090, Korsch XL200, XL400, XL800, may be used. The compression force is preferably between 0.1 to 30 kN.

The controlled release dosage form is preferably an osmotically active dosage form (OROS), in particular a hydromorphone OROS dosage form.

The controlled release dosage form may further be coated with a first coating. The first coating may comprise as coating components cellulose acetate, polyethylene glycol, and any combination thereof. The coating may be applied by spraying a first coating solution onto the tablets or dipping the tablets into a first coating solution. The first coating solution typically comprises the coating components and is an aqueous-acetone solution. Typically, the first coating solution is an aqueous-acetone solution (5/95) with 5 % cellulose acetate and 0.5 % polyethylene glycol.

The controlled release dosage form may further be coated with a second coating on the first coating. The second coating may comprise as coating components hydroxypropyl cellulose, polyethylene glycol, titanium dioxide, ferric oxides, and any combination thereof. The coating may be applied by spraying a second coating suspension onto the tablets or dipping the tablets into a second coating suspension. The second coating suspension solution typically comprises the coating components and is an aqueous suspension. Typically, the second coating is free of the hygroscopic active ingredient. Typically, the first coating solution is an aqueous solution with 10 to 20 % coating components. The dosage form may comprise further coatings on top of the second coating.

### Use of lactose or lactose monohydrate

According to another aspect, the present invention provides the use of lactose or lactose monohydrate as excipient for the granulation of a hygroscopic active ingredient or a salt thereof, particularly of a hygroscopic active ingredient as defined herein, particularly comprised in a controlled release dosage form for oral administration.

According to a further aspect, the present invention provides the use of lactose or lactose monohydrate as excipient during granulation in the production of granulate comprising a hygroscopic active ingredient or a salt thereof, particularly a hygroscopic active ingredient as defined herein; and at least one release controlling hydrophilic polymer, particularly a release controlling hydrophilic polymer as defined herein.

Furthermore, the present invention provides the use of lactose or lactose monohydrate for improving the compressibility of granulate as defined herein. In addition, the present invention provides the use of lactose or lactose monohydrate for improving the flow properties of granulate as defined herein.
In particular, it has been observed that the use of lactose or lactose monohydrate as described herein may result in improved compressibility and flow properties of the obtained granulate.

The lactose or lactose monohydrate may be used in all cases in the form of its monohydrate. Typically, the lactose or the lactose monohydrate is used in an amount of 0.1 to 30 mg for 100 mg granulate.

The lactose or the lactose monohydrate may be used in the form of an aqueous lactose or lactose monohydrate solution, particularly an aqueous solution comprising 1 to 30 wt.-%, more particularly 10 to 20 wt.-%, most particularly 16 to 17 wt.-% lactose or lactose monohydrate.

### Methods for preparing the granulate and the controlled release dosage forms

According to a further aspect, there is provided a method for preparing granulate, comprising granulating a hygroscopic active substance or a salt thereof, particularly as defined herein; at least one release controlling hydrophilic polymer, particularly as defined herein; and lactose or lactose monohydrate. The lactose or lactose monohydrate may be used in the form of an aqueous lactose or lactose monohydrate solution, particularly as defined above.

According to one embodiment, the aqueous lactose or lactose monohydrate solution may be sprayed onto the hygroscopic active substance or a salt thereof and the at least one release controlling hydrophilic polymer, particularly prior to or during the granulation. According to another embodiment, the hygroscopic active substance or a salt thereof and the lactose or lactose monohydrate are dissolved to form an aqueous solution and said aqueous solution is sprayed onto the at least one release controlling hydrophilic polymer, particularly prior to or during the granulation.

According to a further aspect, there is provided a method for preparing granulate, comprising blending a hygroscopic active substance or a salt thereof, particularly as defined herein; and at least one release controlling hydrophilic polymer, particularly as defined herein, spraying an aqueous lactose or lactose monohydrate solution on said blend and granulating said blend with said aqueous lactose or lactose monohydrate solution. Other components selected from diluents, lubricants, colorants, flavorants, glidants, or any combination thereof, may be added during the blending and/or after the granulation and/or during the granulation.

According to all aspects and embodiments, the granulation may be a granulation selected from wet granulation and fluid bed granulation. Fluid bed granulation is particularly preferred.

In the methods as described herein, m · A g of the hygroscopic active ingredient or a salt thereof may be used, wherein A is from 5 to 20, particularly from 10 to 15; and wherein m is from 0.5 to 100, particularly from 1 to 2. Furthermore, m · B g of the at least one release controlling hydrophilic polymer may be used, wherein B is from 70 to 90, particularly from 80 to 85; and wherein m is from 0.5 to 100, particularly from 1 to 2. In addition, m · C g of lactose or lactose monohydrate may be used in the methods as described herein, wherein C is from 5 to 15 g, particularly from 7 to 10 g; and wherein m is from 0.5 to 100, particularly from 1 to 2.

The lactose or the lactose monohydrate may be used in the form of an aqueous lactose or lactose monohydrate solution, particularly an aqueous solution comprising 1 to 30 wt.-%, more particularly 10 to 20 wt.-%, most particularly 16 to 17 wt.-% lactose or lactose monohydrate.

According to a further aspect, there is provided a method for preparing granulate, wherein the granulate comprises a hygroscopic active substance or a salt thereof, particularly as defined herein; at least one release controlling hydrophilic polymer, particularly as defined herein; and lactose or lactose monohydrate,

According to a further aspect, there is provided a method for preparing a controlled release dosage form for oral administration, wherein the controlled release dosage form comprises granulate as defined herein. Typically, the method comprises compressing the granulate to form the controlled release dosage form, which is typically in the form of a tablet. For compressing, a rotary press or a multi-layer rotary press, such as Fette 1200, Fette 3090, Korsch XL200, XL400, XL800, may be used. The compression force is preferably between 0.1 to 30 kN.

According to a further aspect, the present invention relates to a controlled release dosage form for oral administration obtainable by the methods as defined herein.

### Granulate obtainable by methods as defined herein

According to a further aspect of the present invention, there is provided granulate obtainable by a method comprising or consisting of granulating a hygroscopic active substance or a salt thereof, particularly as defined herein; at least one release controlling hydrophilic polymer, particularly as defined herein; and lactose or lactose monohydrate.

According to another aspect, there is provided granulate obtainable by a method comprising or consisting of granulating a hygroscopic active substance or a salt thereof, particularly as defined herein; at least one release controlling hydrophilic polymer, particularly as defined herein; and an aqueous lactose or lactose monohydrate solution.

According to one embodiment, the aqueous lactose or lactose monohydrate solution is typically sprayed onto the hygroscopic active substance or a salt thereof and the at least one release controlling hydrophilic polymer, particularly prior to or during the granulation.

According to another embodiment of the methods as described above, the hygroscopic active substance or a salt thereof and the lactose or lactose monohydrate are dissolved to form an aqueous solution and wherein said aqueous solution is sprayed onto the at least one release controlling hydrophilic polymer, particularly prior to or during the granulation.

According to yet another aspect, there is provided granulate obtainable by a method comprising or consisting of blending a hygroscopic active substance or a salt thereof, particularly as defined herein; and at least one release controlling hydrophilic polymer, particularly as defined herein; spraying an aqueous lactose or lactose monohydrate solution on said blend and granulating said blend with said aqueous lactose or lactose monohydrate solution. Typically, other components selected from diluents, lubricants, colorants, flavorants, glidants, or any combination thereof, are added during the blending and/or after the granulation and/or during the granulation. According to all aspects and embodiments, the granulation may be a granulation selected from wet granulation and fluid bed granulation. Fluid bed granulation is particularly preferred.

The granulate typically comprises the hygroscopic active ingredient or a salt thereof, 30 to 95 mg of at least one release controlling hydrophilic polymer, in 100 mg of the granulate; and 0.5 to 30 mg lactose or lactose monohydrate, in 100 mg of the granulate. Typically, 100 mg granulate comprises 0.1 to 30 mg of the hygroscopic active ingredient or a salt thereof. The lactose or lactose monohydrate is typically used in the form of an aqueous lactose or lactose monohydrate solution, particularly an aqueous solution comprising 1 to 30 wt.-%, more particularly 10 to 20 wt.-%, most particularly 16 to 17 wt.-% lactose or lactose monohydrate. The granulate may be used for preparing a controlled release dosage form for oral administration.

The invention will be more fully understood by references to the following examples. They should not, however, be construed as limiting the scope of the invention. The disclosure of all literature and patent citations mentioned herein are expressly incorporated by reference.

### Examples

### Example 1: Direct compression

Polyethylene oxide and hydromorphone are mixed and directly compressed. The resulting tablets are sticky, show insufficient flow properties, and suffer from inhomogeneities and nest formation.

### Example 2: Fluid bed granulation

2.1 Polyethylene oxide and hydromorphone are granulated with water. The resulting granulate is sticky, shows insufficient flow properties, and is not compressible to tablets.

2.2 Polyethylene oxide and hydromorphone are granulated with a 3 % aqueous solution of polyethylene oxide. The resulting granulate is sticky, shows insufficient flow properties, and is not compressible to tablets.

2.3 Polyethylene oxide and hydromorphone are granulated with a 10 % aqueous solution of polyvinylpyrrolidone. The resulting granulate is sticky, shows insufficient flow properties, and is not compressible to tablets.

2.4 Polyethylene oxide and hydromorphone are granulated with a mixture of water and ethanol. The resulting granulate is sticky, shows insufficient flow properties, and is not compressible to tablets.

2.5 Polyethylene oxide and hydromorphone are granulated with water using a high spray speed. The resulting granulate is sticky, shows insufficient flow properties, and is not compressible to tablets.

2.6 Polyethylene oxide and hydromorphone are granulated with a mixture of water and suspended talcum (1%). The resulting granulate is sticky, shows insufficient flow properties, and is not compressible to tablets.

2.7 Polyethylene oxide and hydromorphone are granulated with a mixture of water and suspended silicon dioxide (1%). The resulting granulate is sticky, shows insufficient flow properties, and is not compressible to tablets.

2.8 Polyethylene oxide and hydromorphone are granulated with a 5 % aqueous solution of lactose. The resulting granulate is not sticky, shows sufficient flow properties, and is compressible to tablets. In sieving measurements, the granulate does not differ from other granulate as obtained from examples 2.1 to 2.7.

2.9 Hydromorphone and lactose are dissolved in water and sprayed onto polyethylene oxide prior to granulation. The resulting granulate is not sticky, shows sufficient flow properties, and is compressible to tablets.

2.10 Lactose is blended with polyethylene oxide and a solution of hydromorphone is sprayed onto the blend prior to the granulation. The resulting granulate is not sticky, shows sufficient flow properties, and is compressible to tablets.

### Example 3: Wet granulation

Not possible, as polyethylene oxide becomes sticky.

As apparent from the examples, direct compression of polyethylene oxide and hydromorphone does not give satisfactory results. The same applies for the granulation of polyethylene oxide and hydromorphone with a 3 % aqueous solution of polyethylene oxide, a 10 % aqueous solution of polyvinylpyrrolidone, a mixture of water and ethanol, a mixture of water and suspended talcum (1%), or a mixture of water and suspended silicon dioxide (1%). However, using lactose or lactose monohydrate as excipient results in improved granulate regarding stickiness, compressibility and flow properties.

### List of references

US 5273758

EP 0769949 B1

The following pages of the description refer to the embodiments of the invention listed as separate items:
1. Granulate comprising:
   - a hygroscopic active ingredient or a salt thereof,
   - 30 to 95 mg of at least one release controlling hydrophilic polymer, in 100 mg of the granulate; and
   - 0.5 to 30 mg lactose or lactose monohydrate, in 100 mg of the granulate.
2. The granulate of item 1 comprising 0.1 to 30 mg of the hygroscopic active ingredient or a salt thereof, in 100 mg of the granulate.
3. The granulate of item 1 or 2, wherein the hygroscopic active ingredient is a pharmaceutical ingredient.
4. The granulate of any of the preceding items, wherein the hygroscopic active ingredient is selected from hydromorphone, hydromorphone salt, methamphetamine, methamphetamine salt, etoricoxib, etoricoxib salt, and any combination thereof, and is particularly hydromorphone or hydromorphone salt.
5. The granulate of any of the preceding items, wherein the hygroscopic active ingredient is present in the form of a salt, particularly in the form of the hydrochloride, sulfate, trifluoracetate, mucate, oleate, acetate, phosphate, or bitartrate salt, or any combination thereof, and is particularly hydromorphone hydrochloride.
6. The granulate of any of the preceding items, comprising 40 to 95 mg, particularly 50 to 95 mg, of at least one release controlling hydrophilic polymer, in 100 mg of the granulate.
7. The granulate of any of the preceding items, wherein the at least one release controlling hydrophilic polymer is an osmotically effective polymer.
8. The granulate of any of the preceding items, wherein the at least one release controlling hydrophilic polymer is selected from polyethylene oxide, cellulose ether, hydroxypropyl methylcellulose, hydroxypropyl cellulose, polyvinyl alcohol, methylcellulose, polyvinylpyrrolidone, or any combination thereof, and is preferably polyethylene oxide.
9. The granulate of item 8, wherein the at least one release controlling hydrophilic polymer has an average molecular weight measured by rheological measurement of 100.000 to 7.000.000 g/mol.
10. The granulate of any of the preceding items, wherein the lactose is present in the form of its monohydrate.
11. The granulate of any of the preceding items, comprising 0.5 to 25 mg, particularly from 0.5 to 20 mg lactose or lactose monohydrate, in 100 mg of the granulate.
12. The granulate of any of the preceding items, further comprising components selected from diluents, lubricants, colorants, glidants, or any combination thereof.
13. A controlled release dosage form for oral administration comprising the granulate of any of items 1 to 12.
14. The controlled release dosage form of item 13, wherein the dosage form is in the form of a tablet.
15. The controlled release dosage form of item 13 or 14, wherein the granulate has been compressed to form the tablets.
16. The controlled release dosage form of any of the preceding items, wherein the tablet is coated with a first coating.
17. The controlled release dosage form of item 16, wherein the tablet is coated with a second coating on the first coating.
18. Use of lactose or lactose monohydrate as excipient for the granulation of a hygroscopic active ingredient or a salt thereof, particularly of a hygroscopic active ingredient as defined in any of items 3 to 5.
19. Use of lactose or lactose monohydrate as excipient during granulation in the production of granulate comprising:
   - a hygroscopic active ingredient or a salt thereof, particularly a hygroscopic active ingredient as defined in any of items 3 to 5;
   - at least one release controlling hydrophilic polymer, particularly a release controlling hydrophilic polymer as defined in any of items 7 to 9.
20. Use of lactose or lactose monohydrate for improving the compressibility of granulate comprising:
   - a hygroscopic active ingredient or a salt thereof, particularly a hygroscopic active ingredient as defined in any of items 3 to 5;
   - at least one release controlling hydrophilic polymer, particularly a release controlling hydrophilic polymer as defined in any of items 7 to 9.
21. Use of lactose or lactose monohydrate for improving the flow properties of granulate comprising:
   - a hygroscopic active ingredient or a salt thereof, particularly a hygroscopic active ingredient as defined in any of items 3 to 5;
   - at least one release controlling hydrophilic polymer, particularly a release controlling hydrophilic polymer as defined in any of items 7 to 9.
22. The use of any of items 18 to 21, wherein the lactose is used in the form of its monohydrate.
23. The use of any of items 18 to 22, wherein the granulate comprises 0.1 to 30 mg of the hygroscopic active ingredient or a salt thereof, in 100 mg of the granulate; and/or 30 to 95 mg of at least one release controlling hydrophilic polymer, in 100 mg of the granulate.
24. The use of any of items 18 to 23, wherein the lactose or the lactose monohydrate is used in an amount of 0.5 to 30 mg for 100 mg of the granulate.
25. The use of any of items 18 to 24, wherein the lactose or the lactose monohydrate is used in the form of an aqueous lactose or lactose monohydrate solution, particularly an aqueous solution comprising 1 to 30 wt.-%, more particularly 10 to 20 wt.-%, most particularly 16 to 17 wt.-% lactose or lactose monohydrate.
25. Method for preparing granulate, comprising granulating
   a hygroscopic active substance or a salt thereof, particularly as defined in any of items 3 to 5;
   at least one release controlling hydrophilic polymer, particularly as defined in any of items 7 to 9;
   and lactose or lactose monohydrate.
26. Method for preparing granulate, comprising granulating
   a hygroscopic active substance or a salt thereof, particularly as defined in any of items 3 to 5;
   at least one release controlling hydrophilic polymer, particularly as defined in any of items 7 to 9;
   and an aqueous lactose or lactose monohydrate solution.
27. The method of item 26, wherein the aqueous lactose or lactose monohydrate solution is sprayed onto the hygroscopic active substance or a salt thereof and the at least one release controlling hydrophilic polymer, particularly prior to or during the granulation.
28. The method of item 25 or 26, wherein the hygroscopic active substance or a salt thereof and the lactose or lactose monohydrate are dissolved to form an aqueous solution and wherein said aqueous solution is sprayed onto the at least one release controlling hydrophilic polymer, particularly prior to or during the granulation.
29. Method for preparing granulate, comprising
   blending a hygroscopic active substance or a salt thereof, particularly as defined in any of items 3 to 5; and at least one release controlling hydrophilic polymer, particularly as defined in any of items 7 to 9;
   spraying an aqueous lactose or lactose monohydrate solution on said blend and granulating said blend with said aqueous lactose or lactose monohydrate solution.
30. The method of item 29, wherein during the blending and/or after the granulation and/or during the granulation components selected from diluents, lubricants, colorants, flavorants, glidants, or any combination thereof, are added.
31. The method of any of items 25 to 30, wherein the granulation is a fluid bed granulation.
32. The method of any of items 25 to 31 using m · A g of the hygroscopic active ingredient or a salt thereof, wherein A is from 5 to 20, particularly from 10 to 15; and wherein m is from 0.5 to 3, particularly from 1 to 2.
33. The method of any of items 25 to 32 using m · B g of at least one release controlling hydrophilic polymer, wherein B is from 70 to 90, particularly from 80 to 85; and wherein m is from 0.5 to 3, particularly from 1 to 2.
34. The method of any of items 25 to 33 using m · C g of lactose or lactose monohydrate, wherein C is from 5 to 15 g, particularly from 7 to 10 g; and wherein m is from 0.5 to 3, particularly from 1 to 2.
35. The method of any of items 25 to 34 using the lactose or lactose monohydrate in the form of an aqueous lactose or lactose monohydrate solution, particularly an aqueous solution comprising 1 to 30 wt.-%, more particularly 10 to 20 wt.-%, most particularly 16 to 17 wt.-% lactose or lactose monohydrate.
36. Method for preparing granulate, wherein the granulate comprises
   a hygroscopic active substance or a salt thereof, particularly as defined in any of items 3 to 5;
   at least one release controlling hydrophilic polymer, particularly as defined in any of items 7 to 9;
   and lactose or lactose monohydrate,
37. Method for preparing a controlled release dosage form for oral administration, wherein the controlled release dosage form comprises the granulate of any of items 1 to 12.
38. The method of item 37, comprising compressing the granulate.
39. Controlled release dosage form for oral administration obtainable by a method of item 37 or 38.
40. Granulate obtainable by a method comprising or consisting of granulating
   a hygroscopic active substance or a salt thereof, particularly as defined in any of items 3 to 5;
   at least one release controlling hydrophilic polymer, particularly as defined in any of items 7 to 9;
   and lactose or lactose monohydrate.
41. Granulate obtainable by a method comprising or consisting of granulating
   a hygroscopic active substance or a salt thereof, particularly as defined in any of items 3 to 5;
   at least one release controlling hydrophilic polymer, particularly as defined in any of items 7 to 9;
   and an aqueous lactose or lactose monohydrate solution.
42. The granulate of item 41, wherein the aqueous lactose or lactose monohydrate solution is sprayed onto the hygroscopic active substance or a salt thereof and the at least one release controlling hydrophilic polymer, particularly prior to or during the granulation.
43. The granulate of item 40 or 41, wherein the hygroscopic active substance or a salt thereof and the lactose or lactose monohydrate are dissolved to form an aqueous solution and wherein said aqueous solution is sprayed onto the at least one release controlling hydrophilic polymer, particularly prior to or during the granulation.
44. Granulate obtainable by a method comprising or consisting of blending a hygroscopic active substance or a salt thereof, particularly as defined in any of items 3 to 5; and at least one release controlling hydrophilic polymer, particularly as defined in any of items 7 to 9;
   spraying an aqueous lactose or lactose monohydrate solution on said blend and granulating said blend with said aqueous lactose or lactose monohydrate solution.
45. The granulate of item 44, wherein during the blending and/or after the granulation and/or during the granulation components selected from diluents, lubricants, colorants, flavorants, glidants, or any combination thereof, are added.
46. The granulate of any of items 40 to 45, wherein the granulation is a fluid bed granulation.
47. The granulate of any of items 40 to 46 comprising 0.1 to 30 mg of the hygroscopic active ingredient or a salt thereof, in 100 mg of the granulate.
48. The granulate of any of items 40 to 47 comprising 30 to 95 mg of at least one release controlling hydrophilic polymer, in 100 mg of the granulate.
49. The granulate of any of items 40 to 48 comprising and 0.5 to 30 mg lactose or lactose monohydrate, in 100 mg of the granulate.
50. The granulate of any of items 40 to 49 using the lactose or lactose monohydrate in the form of an aqueous lactose or lactose monohydrate solution, particularly an aqueous solution comprising 1 to 30 wt.-%, more particularly 10 to 20 wt.-%, most particularly 16 to 17 wt.-% lactose or lactose monohydrate.
51. Use of the granulate of any of items 40 to 50 for preparing a controlled release dosage form for oral administration.

## Claims

1. Granulate comprising:
- a hygroscopic active ingredient or a salt thereof,
- 30 to 95 mg of at least one release controlling hydrophilic polymer, in 100 mg of the granulate; and
- 0.5 to 30 mg lactose or lactose monohydrate, in 100 mg of the granulate.

2. The granulate of claim 1, comprising 0.1 to 30 mg of the hygroscopic active ingredient or a salt thereof, in 100 mg of the granulate.

3. The granulate of claim 1 or 2, wherein the hygroscopic active ingredient is selected from hydromorphone, hydromorphone salt, methamphetamine, methamphetamine salt, etoricoxib, etoricoxib salt, and any combination thereof.

4. The granulate of any of the preceding claims, wherein the at least one release controlling hydrophilic polymer is selected from polyethylene oxide, cellulose ether, hydroxypropyl methylcellulose, hydroxypropyl cellulose, polyvinyl alcohol, methylcellulose, polyvinylpyrrolidone, or any combination thereof.

5. Use of lactose or lactose monohydrate as excipient for the granulation of a hygroscopic active ingredient or a salt thereof.

6. Use of lactose or lactose monohydrate for improving the compressibility and/or the flow properties of a granulate comprising:
- a hygroscopic active ingredient or a salt thereof, and
- at least one release controlling hydrophilic polymer.

7. Method for preparing a granulate, comprising granulating
a hygroscopic active substance or a salt thereof;
at least one release controlling hydrophilic polymer;
and lactose or lactose monohydrate or an aqueous lactose or lactose monohydrate solution.

8. The method of claim 7, wherein the aqueous lactose or the lactose monohydrate solution is sprayed onto the hygroscopic active substance or a salt thereof and the at least one release controlling hydrophilic polymer.

9. The method of claim 7, wherein the hygroscopic active substance or a salt thereof and the lactose or the lactose monohydrate are dissolved to form an aqueous solution and wherein said aqueous solution is sprayed onto the at least one release controlling hydrophilic polymer.

10. The method of any of claims 7 to 9, wherein the granulation is a fluid bed granulation.

11. Granulate obtainable by a method comprising or consisting of granulating a hygroscopic active substance or a salt thereof;
at least one release controlling hydrophilic polymer;
and lactose or lactose monohydrate or an aqueous lactose or lactose monohydrate solution.

12. The granulate of claim 11, wherein the granulation is a fluid bed granulation.

13. A controlled release dosage form for oral administration comprising the granulate of any of claims 1 to 4, 11 and 12.

14. Method for preparing a controlled release dosage form for oral administration, wherein the controlled release dosage form comprises the granulate of any of claims 1 to 4, 11 and 12.

15. Use of the granulate of any of claims 1 to 4, 11 and 12 for preparing a controlled release dosage form for oral administration.
